# EUROPEAN PATENT APPLICATION

(11) **EP 2 992 854 A1**
(43) Date of publication of application: **09.03.2016**
(21) Application number: 14731364.7
(22) Date of filing: 02.05.2014
(51) Int. Cl.: A61D 3/00, H05B 1/02, H05B 3/28, A61G 13/00, A61G 13/10, A61G 13/12

(54) **WORK SURFACE WITH CONTROLLED HEATING ZONE FOR MAINTAINING BODY TEMPERATURE IN ANIMALS**

(30) Priority: 03.05.2013 PT 10693013
(71) Applicant: Laborial Soluções Para Laboratório S.A., 4475-132 Maia (PT); Universidade de Trás-os-Montes e Alto Douro, 5000-911 Vila Real (PT)
(72) Inventor: MARQUES ANTUNES, Luís Miguel Joaquim, P-5001-801 Vila Real (PT); MOITA GORGULHO SOARES BRANCO, José Carlos, P-4470-145 Maia (PT)
(74) Representative: Ferreira, Maria Silvina
(86) International application number: PCT/IB2014/061153
(87) International publication number: WO 2014/178026

(57) **Abstract**

Work surface with a heating area for maintaining body temperature in animals, characterised for comprising: top plate; supplementary plate; heating element enclosed in a defined area of the work space; enclosure for the heating element and control system; feedback control system comprising a temperature probe and temperature controller. In a preferred embodiment, the top plate is made of glass or other non-porous, rigid material; the supplementary plate is made of phenolic resin, glass or other non-absorbent, non-porous, rigid material; the heating element is an electrical resistor; the enclosure is substantially sealed in order to protect the heating element and control system; the temperature controller is PID. In a preferred embodiment, the supplementary plate is placed under the top plate and the heating element is positioned under the top plate in a recessed area cut into the supplementary plate.

## Description

### Technical Field of the Invention

This invention consists of a work surface with a controlled heated area that maintains the animal's body temperature, particularly during surgery under anaesthesia, for use in animal facilities, veterinary clinics, veterinary hospitals and animal research centres.

### Background of the Invention

Hypothermia induced by anaesthesia and surgery can alter physiological processes, prolong the recovery from anaesthesia, or even cause death (Flecknell, 1996; Arras et al, 2001; Rembert et al, 2004). All animals, and rodents in particular, are likely to suffer from hypothermia because they have a large surface area in relation to their low body mass (Flecknell, 1996). The result is a rapid drop of the core body temperature during general anaesthesia. Therefore, the use of heating devices to prevent excessive heat loss during anaesthesia and surgical procedures is recommended.

Heating devices most commonly used in anaesthetic/surgical practices include circulating warm water blankets, heat lamps and electric heating blankets. These systems prevent hypothermia by supplying a continuous source of heat placed under the animal. The use of these devices requires constant supervision in order to avoid thermal injury and iatrogenic hyperthermia due to the use of such devices. These systems are based on a feedback control system, i.e., the control is based on the measurement of the animal's body temperature. However, some of the systems available in the market are based on the application of electrical resistors, which normally do not allow for a homogeneous diffusion of heat. In this context, it was recently shown that some devices may cause burns and compromise the animal's wellbeing (Antunes et al, 2008), as well as change experimental results obtained with animals when these are used as animal models.

### References

Antunes LM, Pinto ML, Silva AM and Alves H (2008). Accidental ear burns following anaesthesia in mice. Scandinavian Journal of Laboratory Animal Science, 35 (4), 299-301.
Arras M, Autenried P, Rettich A, Spaeni D and Rülicke T (2004). Optimisation of intraperitoneal injection anaesthesia in mice: drugs, dosages, adverse effects, and anaesthesia depth. Comparative Medicine, 51(5), 443-56.
Flecknell PA (1996). Laboratory animal anaesthesia: a practical introduction for research workers and technicians; London: Academic Press.
Rembert MS, Smith JA and Hosgood G (2004. A comparison of a forced-air warming system to traditional thermal support for rodent microenvironments. Laboratory Animal, 38(1), 55-63.

### General description of the invention

This invention consists of a work surface with a controlled heated area that maintains the body temperature of animals, for use in animal facilities, veterinary clinics, veterinary hospitals and animal research centres.

It comprises a work surface made of glass or material with similar characteristics and a supplementary plate made of phenolic resin, glass or material with similar characteristics; a resistive heating element and an enclosure for the temperature control system.

It is intended for use in surgery equipment tables, equipment for the maintenance of animal temperature, recovery rooms, intensive care units and laboratory furniture, with aseptic or easily decontaminated materials, or environments with less restrictions.

The description is of a work surface with a heated area to maintain body temperature in animals, which comprises: top plate; supplementary plate; heating element enclosed in a defined area of the work surface; enclosure for the heating element and control system; feedback control system comprising a temperature probe and temperature controller.

In a preferred embodiment, the top plate is made of non-porous glass or other rigid material; the supplementary plate is made of phenolic resin, glass or other, non-absorbent, non-porous rigid material; the heating element is an electrical resistor; the enclosure is substantially sealed in order to protect the heating element and the control system; the temperature controller is a PID and comprises a solid state relay or an equivalent device.

In a preferred embodiment, the supplementary plate is placed under the top plate and the heating element is positioned under the top plate in a recessed area cut into the complementary plate.

In a preferred embodiment, the top plate has a thickness of 5 to 12mm and the heating element has an output power of 50 to 80W, in one particular embodiment, it is 6-10mm thick with an output power of 40-60W, in a more particular embodiment, it is 8 mm thick with an output power of 40-60W, and these powers are for an area with 0.03m². The output power must be adjusted according to the size of the heating area of the top plate.

In a preferred embodiment, the top plate has a thickness of 5 to 12mm and the heating element has an output power of 1667-2667W/m² in one particular embodiment, it is 6-10mm thick with an output power of 1333-2000W/m², in a more particular embodiment, it is 8 mm thick with an output power of 1333-2000W/m². The output power must be adjusted according to the size of the heating area of the top plate.

The technical solution proposed, a work surface with a controlled heated area to maintain body temperature in animals, allows spreading heat homogeneously, ensuring the temperature is even throughout the entire heating area. The materials chosen for the top surface and for the supplementary plate do not allow the accumulation of concentrated thermal energy pockets that can cause burns in the animal's body.

Systems currently available in the market are not designed to ensure the aseptic conditions required by animal surgery. In this regard, the materials used for the top surface and for the complementary plate allow for the integration of the various components, ensuring the easy disinfection of the heating surface, thus preventing contamination.

This work surface is intended for use at animal facilities, veterinary clinics, veterinary hospitals and animal research centres, allowing to maintain the core temperature of the animal during anaesthetic/surgical practices or recovery, without the risk of burns or thermal injury for animals.

This system also ensures a high level of sterility, given the lack of potential contamination pockets. This surface is also suitable for confined laboratory areas intended for the performing of anaesthetic/surgical procedures on animals, including animal facilities and cleanrooms, due to its sterility characteristics.

In a preferred embodiment, this surface shall have no protrusions or recesses that may potentially accumulate dust, dirt or other sources of contamination, as it is built using non-porous, easily decontaminated materials.

The work surface comprises a glass and a phenolic resin plate with a controlled central heating area that maintains the animal's body temperature. It is characterised by the absence of roughness, cracks or other irregularities, which may constitute a source of contamination. Ideally, at the base of the surface there is an enclosure that houses the electronic devices, contributing to the sterility of the equipment.

The technology for maintaining the body temperature of the recovering animal consists of a resistive heating element that heats up a certain area of the work surface, connected to a feedback control system that monitors the animal's temperature, which has with the following characteristics: The animal's body temperature is monitored by a rectal probe or a probe implanted in the animal, which sends a signal to a controller (ex. PID), with an actuator such as a solid state relay or equivalent device, which is, in turn, connected to the resistive element, providing more or less heat, according to a predetermined range of the animal's body temperature.

Preferably, the enclosure installed in the base houses the entire area of the electronic devices, which are potential sources of contamination.

Both the glass and phenolic resin plates are easily disinfected and decontaminated and are appropriate for use in aseptic environments.

Glass has a thermal capacity that allows for a slow and even distribution of heat.

This equipment can be used on workbenches, fume hoods, laboratory furniture, or other laboratory equipment.

The top glass plate can be made of any materials that are rigid, transparent, non-porous and chemically resistant, such as polycarbonate, laminated glass, glass chemically enriched for added strength, or tempered glass.

The supplementary plate, made of glass or resin, can be made of any materials that are rigid, non-porous and chemically resistant, such as phenolic resin, wood, medium density fibreboard (MDF), high density fibreboard (HDF), polycarbonate, polyester or acrylic and its derivatives, particularly acrylic or polyester agglomerates with inert particles.

The description is of a work surface with a heated area to maintain body temperature in animals, which comprises:
a top plate made of glass or other rigid material with equivalent thermal properties, non-porous and easily disinfected;
supplementary plate made of phenolic resin, glass or other non-absorbent, non-porous and easily disinfected rigid material;
a resistive heating element enclosed in a defined area of the work surface;
enclosure (substantially sealed to protect the resistive element and
control system from contaminating and disinfecting agents usually present in these laboratory environments;
feedback-based control system, consisting of a rectal temperature probe or a probe implanted in the animal, a PID-type controller and a solid-state relay or equivalent device.

In a preferred embodiment, the resistive element has a coating to ensure an even temperature across its entire surface.

In a preferred embodiment, the top glass plate heats evenly across the resistive element's projection area, without any thermal energy concentration pockets that can cause burns to the animal's body.

In a preferred embodiment, the resistive element's control system, which actuates according to the temperature measured by the probe implanted in the animal, works in such a way that the maximum temperature variation in the animal is ± 1°C.

In a preferred embodiment, the top plate is made of glass or polycarbonate and, in a particular embodiment, it is made of laminated-glass, glass chemically enriched for added strength, or tempered glass.

In a preferred embodiment, the supplementary plate is made of glass, resin, phenolic resin, medium density fibreboard (MDF), high density fibreboard (HDF), polycarbonate, polyester or acrylic and its derivatives, particularly acrylic or polyester agglomerates with inert particles.

In a preferred embodiment, the top plate covers the entire work surface, avoiding any gaps or roughness.

A preferred embodiment comprises substantially smooth materials, without any sharp edges, visible holes or empty recesses.

The description also includes a workbench, a fume hood, or a laboratory furniture unit that comprises a work surface such as any of those previously described.

### Description of the Figures

To provide a better explanation of the invention, figures are included representing the preferred embodiments of the invention, which, however, are not intended to limit the object of the invention.

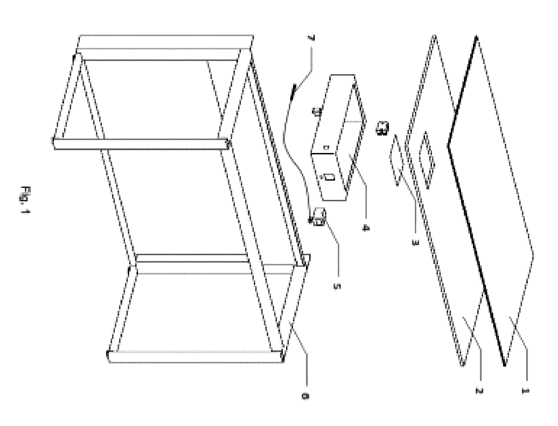

**Figure 1****:** Schematic representation of the equipment's assembly, where:
   (1) represents the glass top plate;
   (2) represents the phenolic resin or glass supplementary plate;
   (3) represents the resistive heating element;
   (4) represents the substantially sealed enclosure that houses the resistive element and the control system;
   (5) represents the control system with probe and temperature controller;
   (6) represents the support structure; and
   (7) represents the temperature probe.

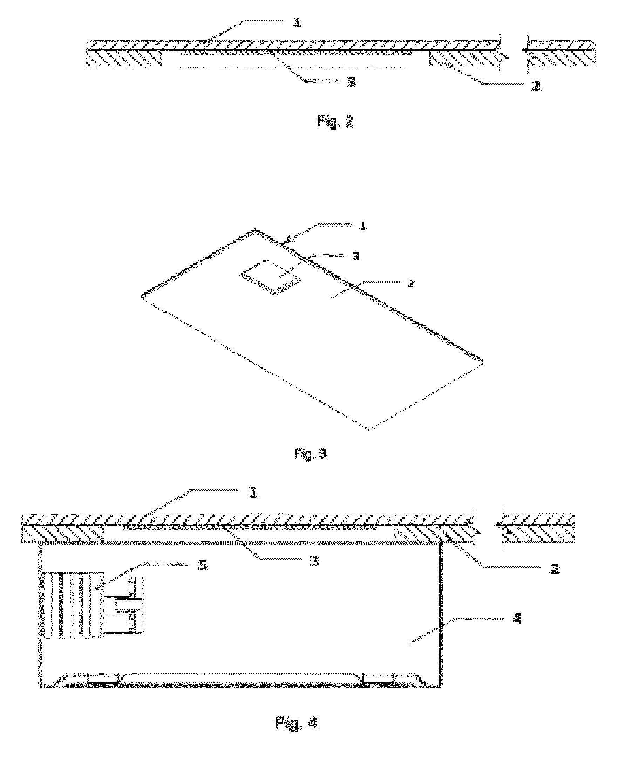

**Figure 2**:Cross-sectional schematic representation of the assembly of the top (1) and supplementary (2) plates and the heating element (3).
**Figure 3****:** Schematic representation of the bottom view of the assembly of the top (1) and supplementary (2) plates and the heating element (3).
**Figure 4****:** Cross-sectional schematic representation of the assembly of the top (1) and supplementary (2) plates and the heating element (3), the enclosure for the heating element and control system (4) and the control system (5).

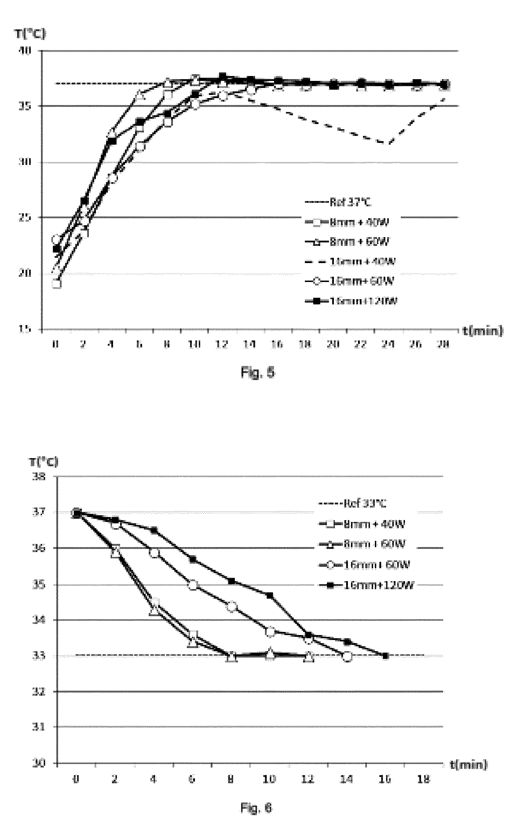

**Figure 5****:** Graphical representation of the evolution of the surface temperature over time, expressed in minutes, for different thicknesses of a glass plate and with different output powers for the heating element placed under the glass plate, when starting the equipment and going from room temperature to a target temperature of 37°C, with the temperature probe resting on the heated surface.
**Figure 6****:** Graphical representation of the evolution of the surface temperature over time, expressed in minutes, for different thicknesses of a glass plate and with different output powers for the heating element placed under the glass plate, when going from a target temperature of 37°C to 33ºC, with the temperature probe resting on the heated surface.

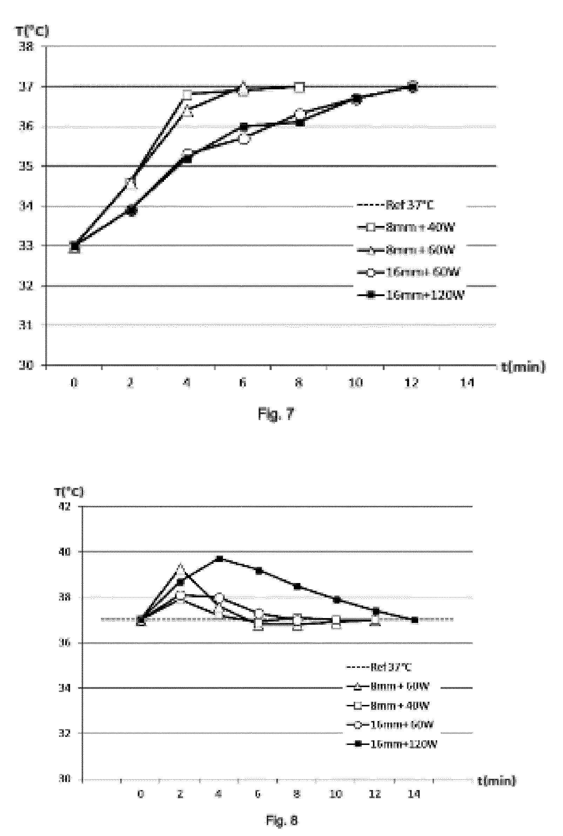

**Figure 7****:** Graphical representation of the evolution of the surface temperature over time, expressed in minutes, for different thicknesses of a glass plate and with different output powers for the heating element placed under the glass plate, when increasing the temperature from 33°C to 37ºC, with the temperature probe resting on the heated surface.
**Figure 8****:** Graphical representation of the evolution of the surface temperature over time, expressed in minutes, for different thicknesses of a glass plate and with different output powers for the heating element placed under the glass, for a target temperature of 37ºC, with the temperature probe being placed away from the heated area for one minute and subsequently placed on the heated surface.

### Detailed description of the invention

**Figure 1** shows the main elements of a preferred embodiment of the equipment, with the top plate (1) made of glass or other rigid, non-porous and easily disinfected material with equivalent thermal properties; the supplementary plate (2), made of phenolic resin, glass or other rigid, non-absorbent, non-porous and easy disinfected material; the resistive element (3) that heats up a delimited area of the work surface; the enclosure (4), substantially sealed to protect the resistive element and control system from contaminating and disinfecting agents usually present in laboratory environments; the feedback-based control system (5), with probe and temperature controller; the support structure (6) and the temperature sensor (7).

**Figure 2** shows a lateral cross-section of the main elements of a preferred embodiment of the equipment, with the top plate (1), made of glass or other rigid, non-porous and easily disinfected material with equivalent thermal properties; the supplementary plate (2), made of phenolic resin, glass or other rigid, non-absorbent, non-porous and easy disinfected material; the resistive element (3) that heats up a delimited area of the work surface.

**Figure 3** shows a bottom view of the main elements of a preferred embodiment of the equipment, with the top plate (1), made of glass or other rigid, non-porous and easily disinfected material with equivalent thermal properties; the supplementary plate (2), made of phenolic resin, glass or other rigid, non-absorbent, non-porous and easy disinfected material; the resistive element (3) that heats up a delimited area of the work surface.

**Figure 4** shows a lateral cross-section of a bottom view of the main elements of a preferred embodiment of the equipment, with the top plate (1), made of glass or other rigid, non-porous and easily disinfected material with equivalent thermal properties; the supplementary plate (2), made of phenolic resin, glass or other rigid, non-absorbent, non-porous and easy disinfected material; the resistive element (3) that heats up a delimited area of the work surface; the enclosure (4), substantially sealed to protect the resistive element and control system from contaminating and disinfecting agents usually present in laboratory environments.

**Figure 5** shows a graphical representation of the evolution of the surface temperature for different thicknesses of a glass plate and with different output powers for the heating element placed under the glass plate, when starting the equipment and going from room temperature to a target temperature of 37°C, with the temperature probe resting on the heated surface, which has an area of 0.03m².

It is clearly noticeable that a thicker glass plate requires more heating power, as, for a glass thickness of 16mm and 40W of output power, the target temperature fails to stabilise, thus rendering this embodiment unfeasible. For the remaining embodiment alternatives, and despite slight variations in the initial room temperature, the target temperature is reached within 8 to 14 minutes.

**Figure 6** shows a graphical representation of the evolution of the surface temperature for different thicknesses of a glass plate and with different output powers for the heating element placed under the glass plate, when going from a target temperature of 37°C to 33ºC, with the temperature probe resting on the heated surface, which has an area of 0.03m².

For a thickness of 16mm, the temperature is much slower to drop, regardless of the power of the heating element, wherein the 16mm + 60W alternative takes 4-6 minutes more and the 16mm + 120W alternative takes 6-8 minutes more to cool down than the 8mm alternatives. A lower thickness, regardless of the output power of the heating element, has the benefit of allowing for faster cooling. This is useful in case of overheating caused, for example, by an external cooling of the temperature probe, as the temperature will drop more quickly to a temperature that prevents causing burns to the animal placed on the heating area of the top surface.

**Figure 7** shows a graphical representation of the evolution of the surface temperature for different thicknesses of a glass plate and with different output powers for the heating element placed under the glass plate, when going from a target temperature of 33°C to 37ºC, with the temperature probe resting on the heated surface, which has an area of 0.03m².

For a thickness of 16mm, the temperature is much slower to rise, regardless of the power of the heating element, wherein 16mm alternatives take 4-6 minutes more to heat up than 8mm alternatives. A lower thickness, regardless of the output power of the heating element, has the benefit of allowing for faster heating. This is useful in case of sudden cooling, for example, due to the top surface being washed with a large quantity of cold liquid, as the temperature will quickly rise to a temperature that prevents situations of hypothermia for the animal placed on the heating area of the top surface.

**Figure 8** shows a graphical representation of the evolution of the surface temperature for different thicknesses of a glass plate and with different output powers for the heating element placed under the glass plate, for a target temperature of 37°C, with the temperature probe being placed away from the heated area for one minute and subsequently placed on the heated surface, which has an area of 0.03m².

For the 16mm thickness, the temperature reaches unwanted peaks, regardless of the output power of the heating element, a variation over 1°C can be enough to cause burns on the more sensitive parts of the animal placed on the heated area of the top surface, such as the ears. A lower thickness is advantageous regardless of the output power of the heating element, as it avoids causing these potentially dangerous temperature spikes.

Considering the embodiments described above, it can be noted that reducing the thickness of the top plate allows for relevantly faster heating and cooling. However, reducing thickness also reduces the sturdiness of the top plate, so the adding of the supplementary plate allows for a structure which is both solid and has an ideal thickness between the heating element and the top surface.

The following claims define further preferred embodiments of this invention.

## Claims

1. Work surface with a heating area for maintaining body temperature in animals, characterised for comprising:
- top plate;
- supplementary plate;
- heating element enclosed in a defined area of the work surface;
- enclosure for the heating element and control system;
- feedback-based control system comprising a temperature probe and a temperature controller.

2. Work surface according to the preceding claim,
- in which the top plate is made of glass or other rigid, non-porous, material;
- in which the complementary plate is made of phenolic resin, glass or other rigid, non-absorbent, non-porous material;
- in which the heating element is resistive;
- in which the enclosure is substantially sealed to protect the resistive element and control system;
- in which the temperature controller is a PID and comprises a solid state relay or equivalent device.

3. A work surface according to any of the preceding claims, in which the supplementary plate is placed under the top plate and the heating element is positioned under the top plate in a recess cut into the supplementary plate.

4. A work surface according to any of the preceding claims, in which the top plate has a thickness of 5-12mm and the heating element has an output power of 1667-2667W/m², in one particular embodiment, a thickness of 6-10mm and an output power of 1333-2000W/m² and in a more particular embodiment, a thickness of 8mm and an output power of 1333-2000W/m².

5. A work surface according to any of the preceding claims, in which the resistive element comprises a coating to even out the temperature throughout its surface.

6. Work surface according to any of the preceding claims, in which the control system for the heating element, controlled by the temperature measured by the probe implanted in the animal, is set to a maximum variation of ±1°C in the animal's body temperature.

7. Work surface according to any of the preceding claims, in which the top plate is made of glass or polycarbonate, particularly laminated glass, glass chemically enriched for added strength, or tempered glass.

8. Work surface according to any of the preceding claims, in which the supplementary plate is made of glass, resin, phenolic resin, medium density fibreboard (MDF), high density fibreboard (HDF), polycarbonate, polyester or acrylic and its derivatives, particularly acrylic or polyester agglomerates with inert particles.

9. Work surface according to any of the preceding claims, in which the top plate covers the entire top working surface, so that no gaps or roughness exist on the top.

10. Work surface according to any of the preceding claims, in which the top plate, the supplementary plate and the respective support structures are made of substantially smooth materials, without any sharp edges, visible holes or empty recesses.

11. Workbench, fume hood, or laboratory furniture unit that comprises a work surface according to the preceding claims.
